Europäisches Patentamt

(19) European Patent Office

Office européen des brevets

(11) Publication number: **0 094 695**
**A1**

(12) # EUROPEAN PATENT APPLICATION

(21) Application number: **83200108.5**

(22) Date of filing: **17.07.79**

(51) Int. Cl.³: **A 61 F 5/46**

---

(30) Priority: **17.07.78 US 924971**

(43) Date of publication of application:
**23.11.83 Bulletin 83/47**

(84) Designated Contracting States:
**FR**

(60) Publication number of the earlier application
in accordance with Art. 76 EPC: **0 016 159**

(71) Applicant: STRICKMAN, Rose
Rd. 1, Lawrence Road
Bridgeton New Jersey 08302(US)

(71) Applicant: FOURNIER, Erick-Pierre
30 Park Avenue
New York, NY 10016(US)

(71) Applicant: STRICKMAN, Melvyn B.
Lawrence Road R.D. 1
Bridgeton, NJ 08302(US)

(72) Inventor: Strickman, Robert L.

deceased(US)

(72) Inventor: Fournier, Erick-Pierre
30 Park Avenue
New York New York 10016(US)

(72) Inventor: Strickman, Melvyn B.
Lawrence Road R.D. 1
Bridgeton New Jersey 08302(US)

(74) Representative: Waxweiler, Jean et al,
Office Dennemeyer S.à.r.l 21-25 Allée Scheffer
Luxembourg(LU)

---

(54) **Disposable contraceptive cervical barrier.**

(57) Disposable contraceptive intravaginal cervical barriers of one, two, three, four, or five layers in the form of standard pessaries with at least one layer impervious to passage of sperm, a medicament such as spermicide, germicide, or an abortion inducing agent in the form of a layer of gel or powder (24), said medicament bein activatable upon contact with an aqeous solution. The medicated pessary solves the problem of the user providing the proper amount and type medicament to the older non-medicated pessary.

FIG.2

EP 0 094 695 A1

DISPOSABLE CONTRACEPTIVE CERVICAL BARRIER

The present invention relates to disposable contraceptive intravaginal barriers. It also describes methods for making these disposable barriers.

Recent incidence of complications with the intrauterine device (IUD) and the contraceptive pill has led women throughout the world to revert to reusable cervical contraceptive pessaries such as the diaphragm and other related cervical pessaries such as the cervical cap, the vault cap, and the vimule. These pessaries, although not as effective as the IUD and the pill preventing pregnancy, have shown no adverse physiological or systematic effects.

Currently available cervical pessaries such as the diaphragm and related cervical pessaries are generally made in the form of a soft rubber cap with a metal spring embedded in the rim. These pessaries present certain disadvantages that operate to discourage their widespread use. Some of these disadvantages are as follows: their effectiveness is substantially lower than the pill, the IUD, and injectable hormones; prior to insertion they must be coated with spermical foam or jelly; after use they must be cleaned and properly stored (this requirement discourage their use especially in areas of the world where water is not readily available); and, because of the cost of spermicides, cervical pessaries can become expensive if used frequently.

Examples of such prior art cervical pessaries are disclosed in CA-A-580311, GB-A-892386 and DE-A-822877.

CA-A-580311 discloses a diaphragm comprising a cup shaped body formed of rubbury material and including an annular circumferential thickened rim and a thin flexible diaphragm portion connected thereto and extending completely across the area within the rim. A continuous nylon spring in the form of a flat ring, cylindrical in form, is wholly enbedded in said rim and extends about said diaphragm.

GB-A-892386 discloses an uterus pessary comprising an outer thin layer of smooth and impervious elastic material, an inner layer of a porous elastic material, and on said inner layer a coating of a chemically active anti-conception agent in admixture with an anti-friction agent having a melting point lower than the normal body temperature.

DE-A-822877 discloses a pessary having a body made of natural or rubber sponge and having one or several liquid impervious intermediate layers and including a recess for receiving the portion of the cervix. This recess also serves to receive an anti-conception ointment which must also be applied to the peripheral edge of the pessary.

All these prior art cervical pessaries have the disadvantages recited hereinbefore.

The object of the invention is to provide a cervical pessary which overcomes the drawbacks of        prior art pessaries, are easy to use, do not discourage wide spread use thereof, are safe in use, and are easy to manufacture.

This object is achieved by a disposable contraceptive intravaginal cervical barrier as claimed in claim 1.

Preferred embodiments of such a contraceptive intravaginal cervical barrier are claimed in claims 2 to 6.

The invention will now be described by way of examples with reference to the accompanying drawings, wherein:

Fig. 1 is a top view of a pessary that can have one, two, three, four or five layers.

Fig. 2 is a side view of Fig. 1 with a quarter section showing a three layer pessary.

Fig. 3 is a side view of Fig. 1 with a quarter section showing a variation of a three layer pessary.

Fig. 4 is a side view of Fig. 1 with a quarter section showing a four layer pessary.

Fig. 5 is a side view of Fig. 1 with a quarter section showing a variation of a four layer pessary.

Fig. 6 is a side view of Fig. 1 with a quarter section showing a five layer pessary.

Fig. 7 is a side view of a flexible ring.

Fig. 8 is a top view of the flexible ring.

Fig. 9 is a side view showing pessary layers in superposition between a male die and a female die.

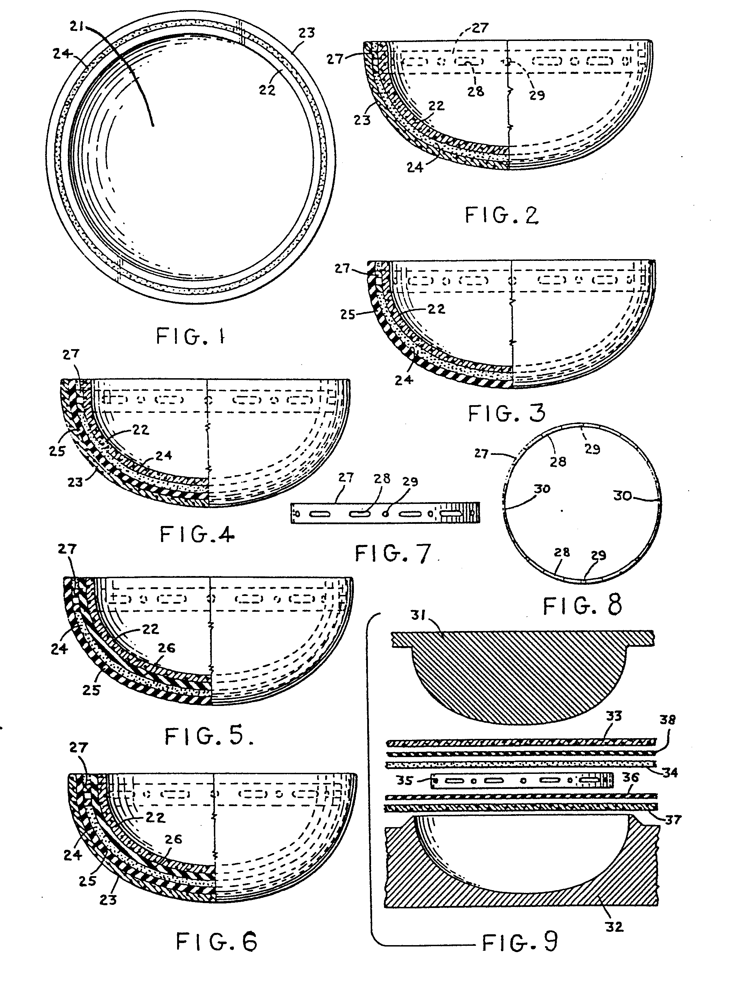

Fig. 1 illustrates a top view of a cervical barrier in accordance with the invention as thermoformed pessary 21. In external contruction, the thermoformed pessaries would include constructions of the various types of intravaginal cervical barriers known in the art, namely, a diaphragm, a vault cap, a vimule, and a cervical cap, together with all the optional variations described here.

The vault cap, sometimes called the Dumascap, generally assumes the shape of a circular bowl. It clings by suction to the vault or roof of the vagina, following the contour of the cervix. It is characterized by a thick rim progressively diminishing to a relatively thin center in the order of no less than 0,32 cm.

The vimule is shaped in the form of a deep, double dome like structure and includes a flanged rim designed to permit the device to be pressed firmly onto the roof of the vagina.

The cervical cap comprises a small, thimble shaped cup designed to block the cervix. It is held in place by suction only.

The diaphragm is held in place by the spring tension of its rim, the woman's vaginal muscle tone, and pubic bone.

Fig. 2 illustrates a quarter section of a three layer construction formed by thermoforming or the dry foam process. Plastic foam layer 22 forms the inner layer of pessary 21, shown as a hollow hemisphere, but with the understanding that this is for purposes of illustration only and can be adapted to other intravaginal cervical barriers. Plastic foam layer 23 forms the outer layer of the pessary. In intimate contact with both layers, or sandwiched between the layers, is a layer of medicaments 24, consisting of spermicides, germicides, or an abortion inducing agent, in the form of a gel with a desirable thickness of 1/2 to 1 millimeter, depending upon the desired degree of concentration. The plastic is preferably polyurethane.

While the medicaments can be easily handled in such a way through the manufacturing process, they can be spontaneously activated at the contact of tap water or any aqeous solution just prior to insertion or just prior to packaging. As they liquify, the medicaments permeate evenly throughout the two layers of polyurethane foam and are held between the open cells. Lowermost section 23 of foam should be desirably cured in such a way that the cells become progressively closed. Bottom surface 23 constitutes a totally impermeable barrier against the passage of sperm from the outside and against the seepage and loss of the medicaments to the outside of the contraceptive barrier.

Fig. 3 is a quarter section illustration of a variation of the three layer construction. Here inner foam layer 22 and medicament layer 24 remain as in Fig. 2, but film layer 25 is placed as the outer layer rather than foam layer 23. Film layer 25 is preferably a highly pliable, resilient film of plastic, preferably polymeric, material that acts as a protective barrier against the diffusion of spermicides into the lower layer of polyurethane foam 22 and as a barrier against the penetration of sperm from the outside. As long as such a barrier is thermoformable, a wide variety of polymeric compositions is deemed suitable, such as natural rubber, latex, polyethylene, polypropylene, polyester, or any number of commercially available coextruded or laminated films.

Fig. 4 is a quarter section of Fig. 1 illustrating a four layer construction. Here a combination of Figs. 2 and 3 is shown. That is, protective film layer 25 is added to Fig. 1 between layers 23 and 24, or, in another sense, foam layer 23 is added as an outer layer to Fig. 3.

Fig. 5 is a quarter section of a second four layer construction. Substantially, flexible top film layer 26 is placed in intimate contact with, that is, is sandwiched between, layer 24 and layer 22. Layer 24 of medicament is then sandwiched between film layers 25 and 26. The top layer of resilient film 26 can be of various kinds in order to allow contact with the water and the medicament for activation, that is, be permeable to water. First, layer 26 can be water soluble. Second, layer 26 can incorporate a plurality of mechanically induced perforations, or tiny holes, to let the water go through. Third, layer 26 can be simply permeable to water simply by virtue of its molecular structure.

Fig. 6 is a cross section of Fig. 1 illustrating a five layer construction. Essentially, it is Fig. 5 with foam layer 23 added as the outside layer to form the fifth layer.

Fig. 7 illustrates a side view of plastic ring 27, which is shown in cross section in Figs. 9,10,11,12,and 13. These contraceptive barriers each incorporate around their rims flat, resilient, plastic ring 27, which optionally forms a series of spaced perforations 28 and 29. These perforations are shown elongated and circular for purposes of illustrations only and can assume a number of varied configurations. Ring 27 is shown in Fig. 2 sandwiched between and joining plastic foam layers 22 and 23, in Figs. 3 and 4 sandwiched between and joining foam layer 22 and film layer 25, and in Figs. 5 and 6 sandwiched between and joining film layers 25 and 26. Perforations 28 and 29 are designed to allow the layers bordering the plastic ring to be heat sealed together through the ring and to permit these three elements to be locked or bound to each other permanently. In effect, the layers bordering the ring are to be fused to each other holes 28 and 29 of the ring.

In order to fold the device in half to facilitate insertion into the vagina, ring 27 can incorporate two diametrically opposed thin points 30 as shown in the top view in Fig. 8. There is of course no restriction to the types of contraceptive barriers that such a structure can be adapted to, that is, it can be applied to a diaphragm, a vimule, a vault cap, or any other medically acceptable intravaginal cervical barrier. The ring can be of any type of flexible and reslient material as long as the proper spring tension can be obtained economically and maintained for the purpose of holding the pessary over the cervix for a one time application.

In the interest of better material handling as well as the elimination of possible adverse effects caused by the combination of pressure and heat exerted by the thermoforming dies, an alternate construction is possible.

Instead of a gel, the layer of medicament can be in the form of a powder or be dehydrated after being applied as a gel. It can also be applied in any fashion. For example, it can be sprayed, or yet sprayed, in liquid or dry form, on

- 6 -

a plastic, on a plastic film substratum. This is provided, of course, that the dry layer 15 be readily soluble on contact with water for activation. The general embodiment would be the same as for Figs. 1,2,3,4,5,6,7, and 8. The powder would lend itself to improved handling over a layer of gel, whose uniformity might be adversely affected during thermoforming.

It is noted that Figs. 2 through 6 can be adapted to include the gripping means in the form of one or several superimposed protruding circumferential ridges (not shown)or in the form of a protrusion (not shown) in the center of internal layer 22.

The medicament, whether gel or powder, can also be time encapsulated for any time release effect, as for example up to 48 hours maximum protection after activation upon contact with an agneous substance.

With regard to the manufacturing of the foregoing thermoformed articles, the various components are assembled and superposed one above the other before being placed between thermoforming male die 31 and female die 32 as illustrated in Fig. 9. The structural elements of the contraceptive barrier illustrated in Fig. 13 include from top to bottom in sequence: inner foam layer 33, preferably polyurethane foam; layer 34 of medicated gel or powder; plastic ring 35; optional layer 36 of polymeric film impermeable to the passage of sperm; and bottom layer 37 of plastic foam, preferably polyurethane. Optional film layer 38 as illustrated in Figs. 12 and 13 can of course be inserted between layers 33 and 34 if a five layer pessary is desired. Likewise, either layer 36 or layer 37 can be optionally withdrawn to make a three layered pessary.

As all of the aforementioned components, except for the plastic ring, are readily thermoformable under a specific temperature range, any die in combination with modern heat will impart to these materials any desired permanent shape. The components of the desired type of pessary are positioned in parallel arrangement between two mating dies. One male die is in the form of a convey punch and one female die is in the form of a reciprocal cavity. As the two heated dies move closer to each other, the polymeric layers become permanently set in the form of a generally hollow shape.

As part of the thermoforming mold, provisions are also made for a circular cutting edge and for an integrated thermal sealing attachment, such as a generator of highfrequency radio energy to weld together the ring and the two foam layers.

With these two added attachments, thermoforming, cutting, and sealing of the article can be effected sequentially in a one step operation that lends itself to fast production cycles and low cost per unit.

After quality control and sterilization, the one time use article can be suitable folded in half along its length, premoistened is so desired (therefore ready for insertion), and packaged in an air tight plastic laminated aluminum foil pouch. The ability of such a disposable contraceptive barrier to be folded and packaged in an unobtrusive way presents a major advantage for the consumer over conventional types that have to be stored flat in a horizontal position.

The preparation of disposable contraceptive intravaginal cervical barriers, manufactured from preferably polyurethane compositions with various additives disposed as an intermediate layer between at least two polyurethane layers has been discussed.

In the manufacturing process, the spermicidal, or contraceptive compositions or formulations can be incorporated as a solid, liquid, cream or ointment, which can be water soluble, partially water soluble, or water insoluble, but is capable of being carried out by compression and release. These diaphragm barriers can be made with a variety of spermicidal agents.

The additive ingredients carried by the intermediate layer are available when required and activated. The ingredients are carried in such a way that their release is made possible by a sqeezing compression effect, and or when water or other fluids entering the cellular structure of the polyurethane layer contact the additive ingredients. The water serves to release these ingredients as soon as contact takes place. The cervical barrier has the "memory of a natural sponge" and returns to its original shape after being sqeezed. This characteristic allows shaped articles made from these compositions to be inserted and removed from body cavities having various anatomical configurations quite easily.

The water, or other liquids, such as internal body fluids, such as internal secretions of the vaginal walls, flows from cell to cell, filling each cell and spilling outward to the adjacent cells. In addition, diffusion of the fluids takes place through the cell walls themselves forming the interstitial matrix. The water, or fluids causes these spermicidal ingredients to flow outward the outer surfaces of the article where they flow onto and contact the inner body cavity surfaces for which intended. Sqeezing and release facilitates this process greatly.

Release can be controlled not only by varying solubility rates of the ingredients, but by varying particle size, and by a combination of the above.

This product, containing a polyurethane component, combined with various active ingredients meets the requirements of a dispensing package. It holds the spermicide compositions within the intermediate layer until use is desired, in a stable form, both chemically, and physically. It then serves to dispense as needed by releasing its contents.

With regard to the previous indications of time encapsulated medicament, an example of time encapsulation or micro-encapsulation is described as follows:

Step I Microcapsules of oils or silicone components are prepared as follows: 1100 grams of gelatin are dissolved in 1.8 liters of water. The water temperature is brought to not more than $54^{\circ}$ to $55^{\circ}$C. 1000 grams of gum arabic are dissolved in 1.8 liters of hot water under the same condition.

Step II The warm solutions from Step I are mixed in a suitable container and heated to exactly $55^{\circ}$C. The solution is stirred and the pH value adjusted to 4.65 by the addition of either 0.1 N NaOH or 0.1 N HCL.

Step III Oil fractions are added as follows: 9 kilos of isopropyl palmitate are added to the mixture with vigorous stirring, the mixture is cooled slowly with stirring, and the mixing is continued until the temperature reaches 10°C.

Step IV The microcapsules are separated from the aqueous solution and dried by treatment with suitable solvents. The size distribution of the microcapsules is determined by the speed by stirring, by the amount of oil, and by the microcapsules' wall components.

The medicament is activated by water before use. Examples of typical spermicidal compositions are given in the following table in which each of the 13 numerals corresponds to a specific formulation with the manufactures's name wherever applicable.

**0094695**

-10-

Formulae Table

(in grams)

| Ingredients | Manufacturer |
| --- | --- |
| Formula | |

| | |
| --- | --- |
| Hypol 2001 | Grace |
| F-202 Prepolymer | Stepan |
| Catalyst | *** |
| H$_2$O Distilled/Deionized | * |
| Silicone Emulsion   Surfactant 2270 | Goldschmidt |
| Nonoxynol 9 | * |
| Dodecaethyleneglycol monolaureate | * |
| Octoxynol | * |
| P.diisobutylphenoxy polyethoxy-<br>  ethanol | * |
| Sodium Carboxymethyl Cellulose | * |
| Methyl Paraben | * |
| Propylene Glycol | * |
| Stearyl Alcohol | * |
| Propyl Paraben | * |
| White Petroleum | * |
| Sodium Lauryl Sulfate | * |
| Koromex Spermicide | Julius<br>Schmidt |
| Delfen | Ortho<br>Pharma |
| Orthogynol | Ortho<br>Pharma |
| Conceptrol | Ortho Pharma |

\*      generic chemicals

\*\*    Koromex can be substituted for orthogynol or conceptrol

\*\*\*  Catalyst composition (100%)  :  H$_2$O 66.6 %; triethanolamine 20.8%;
          triethylenediamine 12.6%

- 11-

Formulae Table   Continued
(in grams)

| 1 | 2 | 3 | 4 | 5 | 6 | 7 | 8 | 9 | 10 |
|---|---|---|---|---|---|---|---|---|----|
| 10 | 10 | | 10 | | | 50 | 50 | 50 | 10 |
| | | | | 10 | 10 | | | | |
| | | | | 0.25 | 0.25 | | | | |
| 10 | 10 | 10 | 10 | | | 55 | 28.5 | 40 | 5 |
| 1 | 1 | 1 | 1 | | | 4 | 4 | 4 | 1 |
| 0.55 | | | | | | | | 0.6 | |
| | 0.55 | | | 0.8 | | | | | |
| | | 0.55 | | | | | | | |
| | | | 0.55 | | 0.8 | 6.6 | | | |
| | | | | | | 1.6 | | | |
| | | | | | | 0.15 | .0125 | | |
| | | | | | | 15 | 6 | | |
| | | | | | | | 12.5 | | |
| | | | | | | | 12.5 | | |
| | | | | | | | .0095 | | |
| | | | | | | | 0.5 | | |

0094695

-12-

Formulae Table    Continued
(in grams)

| 11 | 12 | 13 |
|----|----|----|
| 10 | 10 | 10 |
|    |    |    |
| 5  | 5  | 5  |
| 1  | 1  | 1  |

|   | ** | ** |
|---|----|----|
| 10 |    |    |
|    | 20 |    |
|    |    | 20 |

,

BUREAU
OMPI
WIPO
INTERNATIONAL

0094695

- 13 -

<u>CLAIMS:</u>

1.    A disposable contraceptive intravaginal cervical barrier comprising first substantially circular flexible · member (23) of foam plastic material and impermeable to the passage of sperm, a rim portion unitary with said flexible member cooperating to define a means for achieving a spring tension effect and a medicament, characterized by a second substantially circular flexible member (21) of a foam plastic material and permeable to the passage of water and a layer (24) of said medicament intimately formed between said first and second members (21,23), said medicament being activatable upon contact with an aqueous substance.

2.    The contraceptive barrier of claim 1, characterized in that said foam plastic material is polyurethane.

3.    The contraceptive barrier of claim 1 or 2, characterized in that said medicament is encapsulated for a preset time release period activatable upon contact with an aqueous substance.

4.    The contraceptive barrier of anyone of the claim 1 to 4, characterized in that said medicament is a spermicide, germicide or abortion inducing agent.

FIG. 1

FIG. 2

FIG. 3

FIG. 4

FIG. 7

FIG. 8

FIG. 5.

FIG. 6

FIG. 9

0094695

EUROPEAN SEARCH REPORT

European Patent Office

Application number

EP 83 20 0108

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | GB-A- 21 588 (FICKERT)(1896) <br> * Page 1, lines 23-33; figures * | 1,3,4 | A 61 F 5/46 |
| D,Y | DE-C- 822 877 (GOOSSENS) <br> * Page 2, lines 1-75; figures * | 1,3,4 | |
| A | US-A-3 261 353 (JOHNSON) <br> * Colonne 1, lines 47-59; figures * | 1,2,4 | |
| A | GB-A-1 597 169 (THE PROCTER & GAMBLE COMPANY) | 1,3,4 | |
| D,A | GB-A- 892 386 (RANDSTROM) | 1 | |
| D,A | CA-A- 580 311 (MAKI) | 1 | TECHNICAL FIELDS SEARCHED (Int. Cl. ³) <br><br> A 61 F |

The present search report has been drawn up for all claims

| Place of search THE HAGUE | Date of completion of the search 26-05-1983 | Examiner STEENBAKKER J. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82